# EUROPEAN PATENT APPLICATION

(11) **EP 4 567 306 A1**
(43) Date of publication of application: **11.06.2025**
(21) Application number: 23214659.7
(22) Date of filing: 06.12.2023
(51) Int. Cl.: F16J 15/00, F16J 15/06, H05K 5/06, A61B 50/30

(54) **MULTI-FUNCTION INTERFACE SEALS**

(71) Applicant: Koninklijke Philips N.V., 5656 AG Eindhoven (NL)
(72) Inventor: RAHAMAN, Arif Shameem, 5656 AG Eindhoven (NL); KOSTAKIS, Dimitri George, 5656 AG Eindhoven (NL)
(74) Representative: Philips Intellectual Property & Standards

(57) **Abstract**

Provided herein are multi-function interface seals and devices or components incorporating one or more of such multi-function interface seals. In embodiments, each multi-function interface seal (100) can be formed by a first component (120) having a first surface (122) comprising a multi-function interface (124), and a second component (130) having a second surface (132) comprising a complementary interface (134). When the first and second components are assembled together, the multi-function interface (124) and the complementary interface (134) form one or more openings wherein at least a first and second sealing member can be disposed in order to provide a measure of protection from one or more external factors, such as heat, EMI (electronic magnetic interference), water, dirt, and/or the like.

## Description

### FIELD OF THE INVENTION

The present disclosure relates generally to interface seals for electronic devices, and more specifically to medical devices, employing multi-function interface seals.

### BACKGROUND OF THE INVENTION

Electronic devices usually include a number of seals, gaskets, and/or connecting materials that join two or more components together. This is especially true in medical devices, where external conditions can affect the performance of sensitive equipment. For example, many medical devices will include seals, gaskets, and/or other connecting materials that shield from various external conditions (external thermal conditions, external electrical conditions, external mechanical conditions, external environmental condition, and/or the like). Notably, providing a certain level of protection (e.g., thermal protection, electromagnetic protection, debris protection, etc.) via such seals, gaskets, and/or other connecting materials may be required by regulatory / industry standards. In order to meet these required specifications, a device will include seals, gaskets, and/or other connecting materials at different locations of the device. Nevertheless, the distributed nature of these seals, gaskets, and/or other connecting materials can make assembly and maintenance of such devices difficult.

### SUMMARY OF THE INVENTION

The invention is defined by the independent claims. Advantageous embodiments are defined in the dependent claims.

Described herein are multi-function interface seals that provide multi-functional protection for a given device from undesirable external and/or environmental factors, such as heat, electromagnetic interference, water, debris, and the like. More specifically, the multi-function interface seal provides an all-in-one solution reducing the number of parts and critical interfaces necessary to meet the required specifications and achieve optimum performance.

According to an aspect of the present disclosure, a multi-function interface seal is provided and includes: a first component having a first surface comprising a multi-function interface; a second component having a second surface comprising a complementary interface, wherein the multi-function interface and the complementary interface form at least a first recess and a second recess between the first and second components when the first and second components are assembled together; a first seal configured to be disposed within the first recess when the multi-function interface seal is assembled, wherein the first seal is configured to provide protection from at least a first external source; and a second seal configured to be disposed within the second recess when the multi-function interface seal is assembled, wherein the second seal is configured to provide protection from at least a second external source.

In an embodiment, the first external source includes at least one of an external heat factor, an external electrical factor, an external mechanical factor, and an external environmental factor.

In an embodiment, the first and second recesses formed by the multi-function interface and the complementary interface are adjacent to one another.

In an embodiment, the first and second recesses formed by the multi-function interface and the complementary interface have different cross-sectional dimensions.

In an embodiment, the second external source includes at least one of an external heat factor, an external electrical factor, an external mechanical factor, and an external environmental factor.

In an embodiment, the second external source is different from the first external source.

In an embodiment, the multi-function interface and the complementary interface are formed at least a third recess between the first and second components when the first and second components are assembled together.

In an embodiment, the first recess, second recess, and third recess formed by the multi-function interface and the complementary multi-function interface are adjacent to one another, and the first recess, second recess, and third recess have different cross-sectional dimensions.

In an embodiment, the multi-function interface seal further includes at least a third seal configured disposed within the third recess when the multi-function interface seal is assembled, wherein the third seal provides protection from at least a third external source, the third external source including at least one of an external heat factor, an external electrical factor, an external mechanical factor, and an external environmental factor.

In an embodiment, the third external source is different from the first and second external sources.

According to another aspect of the present disclosure, a medical device comprising a multi-function interface seal according to claims 1-10 is provided, wherein the first component (120) is a printed circuit assembly, and wherein the second component (130) is a mechanical assembly. The medical device can include: a printed circuit assembly having a first surface comprising a multi-function interface; a mechanical assembly having a second surface comprising a complementary interface, wherein the multi-function interface and the complementary interface form at least a first recess and a second recess between the printed circuit assembly and the mechanical assembly when the printed circuit assembly and the mechanical assembly are assembled together; a first seal configured to be disposed within the first recess when the printed circuit assembly and the mechanical assembly are assembled together, wherein the first seal is configured to provide protection to the printed circuit assembly from at least a first external source; and a second seal configured to be disposed within the second recess when the printed circuit assembly and the mechanical assembly are assembled together, wherein the second seal is configured to provide protection to the printed circuit assembly from at least a second external source.

In an embodiment, the medical device further comprises a second multi-function interface seal.

These and other aspects of the invention will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a perspective view of a device having a multi-function interface seal illustrated in accordance with embodiments of the present disclosure.
FIG. 2 is a partial exploded view of a multi-function interface seal illustrated in accordance with embodiments of the present disclosure.
FIG. 3 is a partial exploded view of certain components of a multi-function interface seal illustrated in accordance with embodiments of the present disclosure.
FIG. 4 is a partial perspective view of an assembly device having a multi-function interface seal illustrated in accordance with embodiments of the present disclosure.
FIG. 5 is a side view of a multi-function interface seal illustrated in accordance with embodiments of the present disclosure.
FIG. 6 is a side view of a multi-function interface seal illustrated in accordance with embodiments of the present disclosure.
FIG. 7 is an exploded side view of a second multi-function interface seal illustrated in accordance with embodiments of the present disclosure.
FIG. 8 is a side view of a second multi-function interface seal illustrated in accordance with further embodiments of the present disclosure.

### DESCRIPTION OF EMBODIMENTS

Provided herein are multi-function interface seals and devices employing one or more of such multi-function interface seals. As described, the multi-function interface seals localize two or more seals into the same region of the interface / device while still providing protection from a number of different external and/or environmental factors.

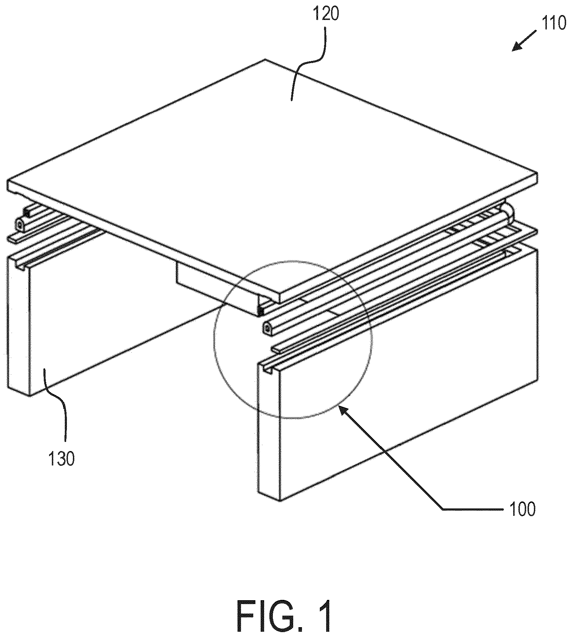

For example, with reference to FIG. 1, an exploded perspective view of a multi-function interface seal 100 is illustrated in accordance with certain aspects of the present disclosure. As shown, the multi-function interface seal 100 may be incorporated into a larger device or assembly 110. The multi-function interface seal 100 is formed between a first component 120 and a second component 130 when the first and second components 120, 130 are assembled together. In embodiments, the multi-function interface seal 100 may extend the entire area where the first and second components 120, 130 could contact one another, or at least a portion of such an area. In the example of FIG. 1, the multi-function interface seal 100 extends around the perimeter of the first component 120 thereby shielding the first component 120 from the second component 130.

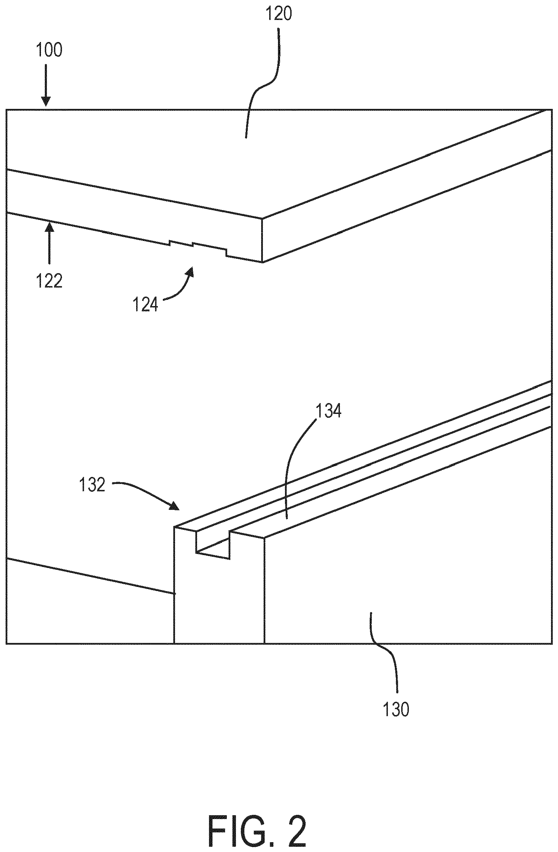

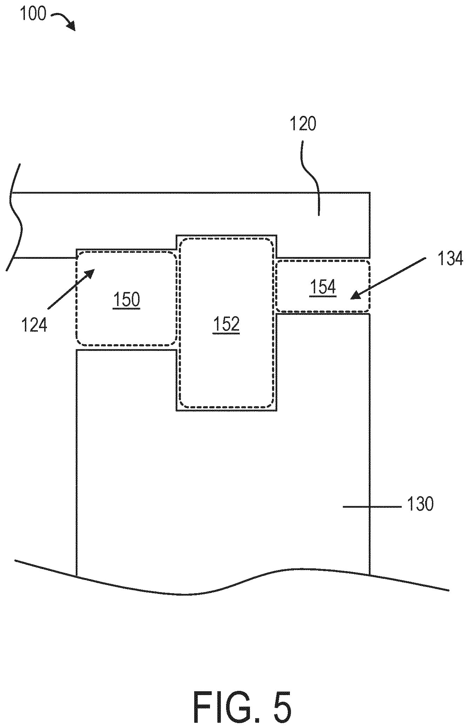

More specifically, with reference to FIG. 2, the first component 120 has a first surface 122 comprising at least a multi-function interface 124, while the second component 130 has a second surface 132 comprising at least a complementary interface 134. In embodiments, the first surface 122 of the first component 120 and the second surface 132 of the second component 130 may oppose one another, as shown in the example of FIG. 2, such that the interfaces 124, 134 form two or more recesses (e.g., recesses 150, 152, 154, etc. as shown in FIG. 5) when the first and second components 120, 130 are assembled together. As discussed in more detail below, the interfaces 124, 134 may form three or more recesses (e.g., recesses 150, 152, 154, etc.) between the first and second surfaces 122, 132 when the first and second components 120, 130 are assembled together.

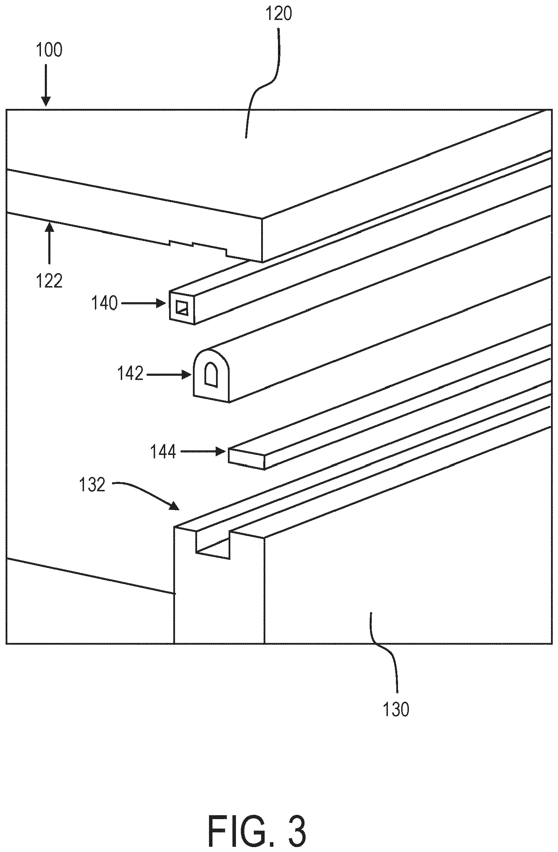

With reference to FIG. 3, the multi-function interface seal 100 further includes two or more sealing members 140, 142, 144 configured to be disposed within the recesses (e.g., recesses 150, 152, 154, etc. as shown in FIG. 5) formed by the interfaces 124, 134 when the first and second components 120, 130 are assembled together. As described herein, each sealing member 140, 142, 144 may independently be a type of seal, gasket, and/or other connecting material that is configured to provide protection from at least one external source. For example, a sealing member 140, 142, 144 may be configured to provide protection from external sources and/or factors including, but not limited to, external heat sources, external electrical sources, external mechanical sources, external environmental sources, and/or the like. Put another way, each of the sealing members 140, 142, 144 should be configured to provide a level of protection against heat, electricity, electromagnetic interference, mechanical forces, liquids or physical debris (e.g., dirt, etc.), and/or the like. In further embodiments, one or more sealing members 140, 142, 144 may be an adhesive sealing member that joins or connects two or more components, such as components 120, 130.

In embodiments, each of the sealing members 140, 142, 144 may provide protection against a different type of external source. For example, in some embodiments, the multi-function interface seal 100 can include a first sealing member 140 configured to provide protection from at least a first external source, and a second sealing member 142 configured to provide protection from at least a second external source. In further embodiments, the multi-function interface seal 100 may include one or more additional sealing members 140, 142, 144, such as at least a third sealing member 144 that is configured to provide protection from at least a third external source.

As shown in FIG. 3, each of the sealing members 140, 142, 144 of the multi-function interface seal 100 may be disposed in recesses (e.g., recesses 150, 152, 154, etc.) such that the sealing members 140, 142, 144 are adjacent to one another. In further embodiments, each of the sealing members 140, 142, 144 of the multi-function interface seal 100 may have different cross-sectional dimensions geared towards the particular external source the respective sealing member is to address. For example, in the example of FIG. 3, the sealing member 140 has a square cross-section with a square hole through its center, the sealing member 142 has a D-shaped cross-section with a D-shaped hole through its center, and the sealing member 144 has a solid rectangular cross-section.

Although a particular combination of sealing members 140, 142, 144 having particular geometries is shown, it should be noted that other combinations of sealing members 140, 142, 144 and/or sealing members 140, 142, 144 having different geometries may be utilized. For example, one or more of the sealing members 140, 142, 144 may be a P-shaped seal, a D-shaped seal, an E-shaped seal, a lip seal, a circular-shaped seal, and/or the like.

The material used to form each of the sealing members 140, 142, 144 utilized in the multi-function interface seal 100 may be different, for example, depending on the intended use. In embodiments, each sealing member 140, 142, 144 may be formed from a rubber material (e.g., neoprene, nitrile, ethylene propylene diene monomer, silicone rubber, styrene butadiene rubber, natural rubber, polyurethane rubber, etc.), fiber material, metal and/or metal alloys, foamed material, an adhesive material (e.g., an acrylic or modified acrylic polymer, a foam tape, a double-sided adhesive tape, a VHB tape, a UHB tape, etc.) and/or any other suitable material, including combinations thereof.

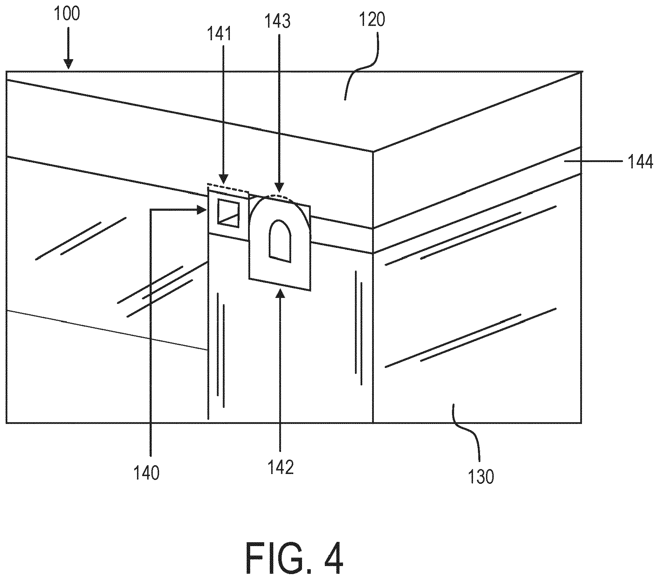

In particular embodiments, each of the sealing members 140, 142, 144 of the multi-function interface seal 100 may require a different level of compression in order to achieve the desired performance (i.e., the desired level of protection from the intended external source(s)). For example, with reference to FIG. 4, the first and second components 120, 130 are assembled together with the sealing members 140, 142, 144 disposed within corresponding recesses (e.g., recesses 150, 152, 154, etc.) formed between the multi-function interface 124 of the first component 120 and the complementary interface 134 of the second component 130. As shown, the sealing members 140, 142 are compressed between the first and second components 120, 130, as indicated by the portions 141, 143. In contrast, the sealing member 144 may not be compressible, or may not be required to be compressed in order to achieve its intended function. As such, in embodiments, the amount by which each sealing member 140, 142, 144 needs to be compressed may be different. Accordingly, in embodiments, the multi-function interface 124 of the first component 120 and the complementary interface 134 of the second component 130 may be designed such that the recesses (e.g., recesses 150, 152, 154, etc.) formed therebetween optimally compress each corresponding sealing member 140, 142, 144. Such designs may accommodate sealing members 140, 142, 144 having different cross-sectional dimensions, and/or geometries as discussed above, for improving the sealing properties of the multi-function interface seal as well as for ease of assembly.

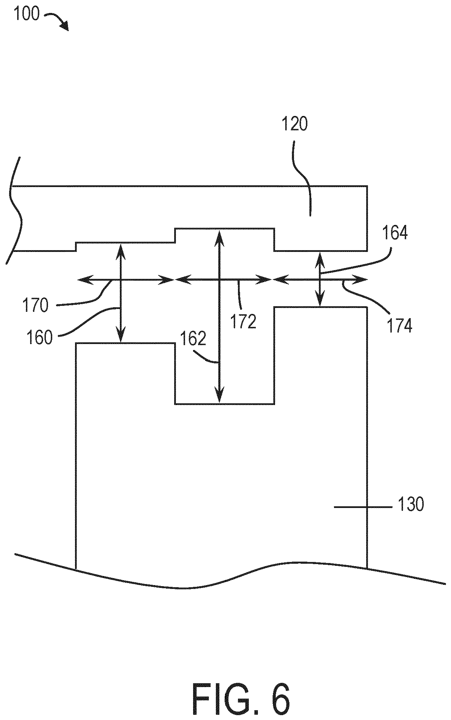

More specifically, with reference to FIG. 5 and FIG. 6, the relationship between the recesses 150, 152, 154 of the multi-function interface seal 100 formed between the first and second components 120, 130 is illustrated. As shown, the first and second components 120, 130 are positioned in an assembled state (with the sealing members 140, 142, 144 not shown), such that the multi-function interface 124 and complementary interface 134 form three distinct recesses or channels 150, 152, 154. In embodiments, each of the recesses or channels 150, 152, 154 of the multi-function interface seal 100 may be adjacent to one another and may have different cross-sectional dimensions. For example, the multi-function interface 124 and the complementary interface 134 may form a first recess 150 having a first height 160 and first width 170, a second recess 152 having a second height 162 and a second width 172, a third recess 154 having a third height 164 and a third width 174, and so on.

Although the recesses or channels 150, 152, 154 as illustrated in FIGS. 5 and 6 each have a rectangular cross-section, it should be noted that the recesses 150, 152, 154 can have other cross-sectional shapes as well. In particular embodiments, one or more of the recesses 150, 152, 154 may be filleted, beveled, chamfered, and/or the like, including combinations thereof.

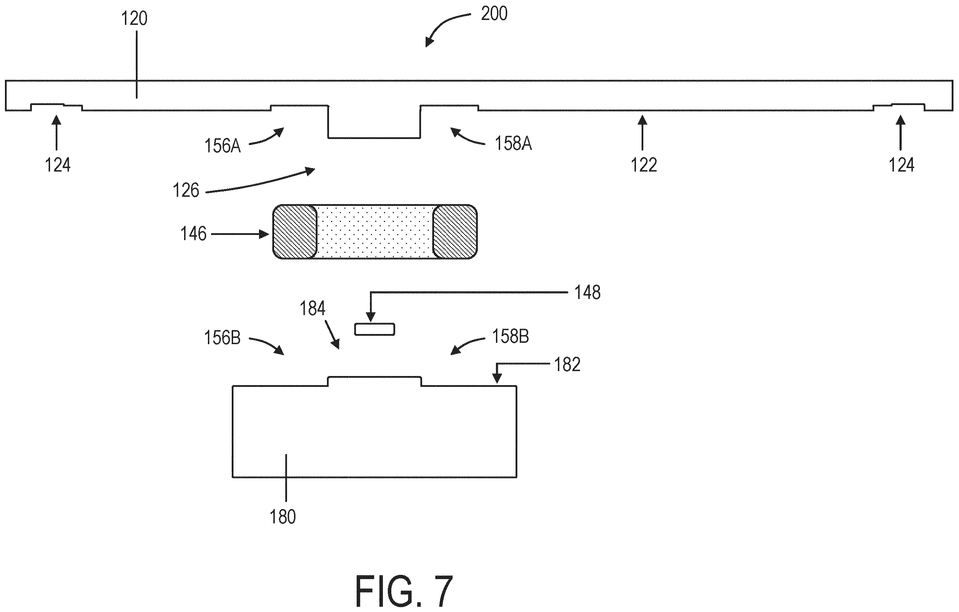

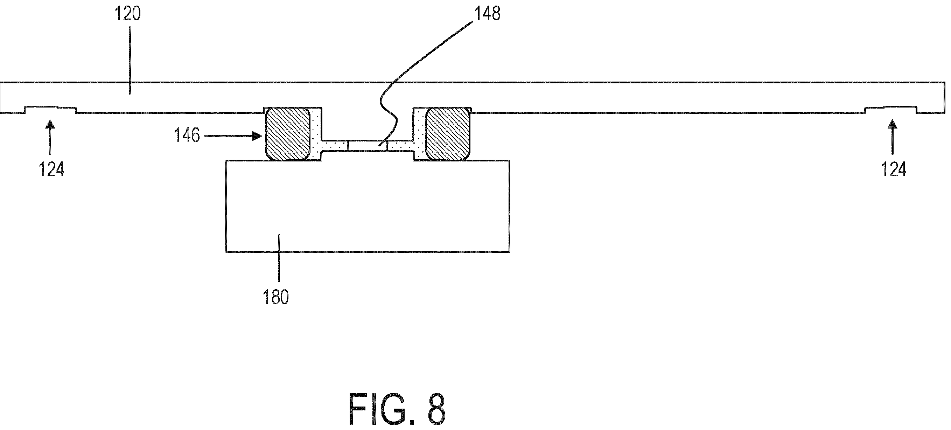

Also provided herein are devices having more than one multi-function interface seal 100. For example, with reference to FIGS. 7 and 8, a second multi-function interface seal 200 is illustrated in accordance with further aspects of the present disclosure. As shown in FIG. 7, the multi-function interface seal 200 is formed between the first surface 122 of the first component 120 and another surface 182 of another component 180. In embodiments, the component 180 may be the same or different from the second component 130. That is, the component 180 may be a part of the second component 130, or may be a separate element not otherwise connected to the second component 130. In particular embodiments, the first surface 122 of the first component 120 has a second multi-function interface 126, while the surface 182 of the component 180 has a complementary interface 184 such that the multi-function interface 126 and the complementary interface 184 form at least a first recess 156A, 156B and a second recess 158A, 158B when the components 120, 180 are assembled together.

In the example of FIG. 7, the second multi-function interface seal 200 further comprises a first sealing member 146 configured to be disposed within the first recess 156A, 156B and the second recess 158A, 158B. In embodiments, the second multi-function interface seal 200 can further comprise one or more additional sealing members, such as sealing member 148, which are also disposed between the surfaces 122, 182 when the components 120, 180 are assembled together as shown in the example of FIG. 8.

As described above with respect to sealing members 140, 142, 144, it should be appreciated that sealing members 146, 148 may similarly having different compositions, different cross-sectional dimensions, and different intended purposes. That is, in particular embodiments, the sealing member 146 may be configured to provide protection from an external source, and sealing member 148 may be configured to provide protection from a different external source.

Referring back to FIG. 1, also provided herein are medical devices or assemblies 110 that incorporate one or more multi-function interface seals 100, 200 in accordance with various aspects of the present disclosure. In particular embodiments, the first component 120 of the medical device / assembly 110 can be, for example and without limitation, a printed circuit board or printed circuit assembly that must be shielded or otherwise protected from one or more external sources or factors. That is, each of the sealing members 140, 142, 144, 146, 148 of the multi-function interface seal 100, 200 of the device 110 can be independently configured to protect the printed circuit assembly 120 from one or more types of external factors, heat, EMI (electronic magnetic interference), water, dirt, and/or the like. In such embodiments, the second component 130 and/or the third component 180 may be a mechanical assembly or housing for the medical device 110.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary will refer to the inclusion of exactly one element of a number or list of elements. In general, the term "or" as used herein shall only be interpreted as indicating exclusive alternatives (i.e. "one or the other but not both") when preceded by terms of exclusivity.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "having," and the like are to be understood to be open-ended, i.e., to mean including but not limited to.

In the claims, any reference signs placed between parentheses shall not be construed as limiting the claim.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A multi-function interface seal (100), comprising:
a first component (120) having a first surface (122) comprising a multi-function interface (124);
a second component (130) having a second surface (132) comprising a complementary interface (134) to the multi-function interface (124), wherein the multi-function interface (124) and the complementary interface (134) form at least a first recess (150) and a second recess (152) between the first and second components (120, 130);
a first sealing member (140) configured to be disposed within the first recess (150), wherein the first sealing member (140) is configured to provide protection from at least a first external source; and
a second sealing member (142) configured to be disposed within the second recess (152), wherein the second sealing member (142) is configured to provide protection from at least a second external source.

2. The multi-function interface seal (100) of claim 1, wherein the first external source includes at least one of an external heat factor, an external electrical factor, an external mechanical factor, and an external environmental factor.

3. The multi-function interface seal (100) of claim 1, wherein the first and second recesses (150, 152) formed by the multi-function interface (124) and the complementary interface (134) are adjacent to one another.

4. The multi-function interface seal (100) of claim 1, wherein the first and second recesses (150, 152) formed by the multi-function interface (124) and the complementary interface (134) have different cross-sectional dimensions.

5. The multi-function interface seal (100) of claim 1, wherein the second external source includes at least one of an external heat factor, an external electrical factor, an external mechanical factor, and an external environmental factor.

6. The multi-function interface seal (100) of claim 5, wherein the second external source is different from the first external source.

7. The multi-function interface seal (100) of claim 1, wherein the multi-function interface (124) and the complementary interface (134) form at least a third recess (154) between the first and second components (120, 130).

8. The multi-function interface seal (100) of claim 7, wherein the first recess (150), second recess (152), and third recess (154) formed by the multi-function interface (124) and the complementary interface (134) are adjacent to one another, and wherein the first recess (150), second recess (152), and third recess (154) have different cross-sectional dimensions.

9. The multi-function interface seal (100) of claim 7, further comprising at least a third sealing member (144) configured to be disposed within the third recess (154), wherein the third sealing member (144) provides protection from at least a third external source, the third external source including at least one of an external heat factor, an external electrical factor, an external mechanical factor, and an external environmental factor.

10. The multi-function interface seal (100) of claim 9, wherein the third external source is different from the first and second external sources.

11. A medical device (110) comprising a multi-function interface seal (100) according to any of claims 1 - 10, wherein the first component (120) is a printed circuit assembly, and wherein the second component (130) is a mechanical assembly.

12. A component (120, 130) for use in a multi-function interface seal (100) according to any of claims 1 - 10, the component having a surface (122, 132) comprising a multi-function interface (124, 134), the multi-function interface (124, 134) having:
a first part configured to be positioned opposite a complementary first part of a complementary interface of another component so as to form a first recess (150), wherein the first recess (150) is configured for receiving a first sealing member (140) configured to provide protection from at least a first external source; and
a second part configured to be positioned opposite a complementary second part of the complementary interface of the other component so as to form a second recess (152), wherein the second recess (152) is configured for receiving a second sealing member (142) configured to provide protection from at least a second external source.
